Europäisches Patentamt

European Patent Office

Office européen des brevets

(19)

(11) Publication number: **0 177 075**
**A2**

(12) **EUROPEAN PATENT APPLICATION**

(21) Application number: **85201351.5**

(22) Date of filing: **23.08.85**

(51) Int. Cl.⁴: **A 63 F 9/22**

(30) Priority: **30.08.84 IT 8280984**

(43) Date of publication of application:
**09.04.86 Bulletin 86/15**

(84) Designated Contracting States:
**AT BE CH DE FR GB LI LU NL SE**

(71) Applicant: **COMPAGNIA INVESTIMENTI E PARTECIPAZIONI AZIONARIE CIPA S.p.A.**
**Via G. Fara 39**
**I-20124 Milano(IT)**

(72) Inventor: **Ugo, Licinio**
**Via Valdirivo 25**
**I-34132 Trieste(IT)**

(72) Inventor: **De Bortoli, Vinicio**
**34019 Sistiana 15M**
**Trieste(IT)**

(74) Representative: **De Carli, Erberto et al,**
**ING. BARZANO' & ZANARDO MILANO S.p.A. Via Borgonuovo, 10**
**I-20121 Milano(IT)**

(54) **Electronic system prearranged to replace in the electronic games the existing manual controls with equivalent controls directly commanded by the brain.**

(57) The present invention consists in a multi-functional electronic device, having the purpose, when added to an electronic game, of replacing in its base programme the voluntary manual interference for the playing of the same game, with a voluntary interference of not manual character, but directly drawn from cerebral electrical impulses, thus rending the game directly influenceable by the will of the player.

EP 0 177 075 A2

## Disclosure

Many and various electronic games exist presently, both of individual character and for more players, which are based on the use of microprocessors and of electronic memories, into which programme softwares have been introduced to the purpose of obtaing variable images on a T.V. screen, manually controllable, by means of levers, knobs or push-buttons, to obtain amusing and competitive effects. Clearly, in order to participate in the games, the player inserts himself into the programme from the outside, by means of these prearranged manual controls, modifying the course of the programmed sequences, and interfering into the image building, with the aim of causing the result of the game to be in his favour.

It is moreover known that by means of the encephalographs it is possible to pick up, by means of electrodes applied on to a man's head, a variable set of electrical impulses, having some prevailing frequency values, and in particular different according to the activity state of the brain.

The range of frequencies occurring is normally quite narrow, and may range from 35 to 5 cycles/second, and depends on the cerebral activity, which may vary from a state of hyperexcitation up to a sleep state, passing through intermediate levels. Although the frequency variations occur in man unconsciously and instinctively, it is however possible to influence, with some practice, the passage from une value to another one quite easily.

The present invention takes hence advantage of this latter possibility of human mind to voluntarily intervene to modify the above mentioned frequencies, also taking

2.

**0177075**

their variation as a basis for controlling a pre-constituted electronic device capable of building such adapted and encoded electrical signals as to be suitable to be
introduced into the games instead of the manually variable
electrical components present in the same games.

While considering that the present disclosure is
given to exemplifying only and not limitative purposes,
generally as a complement to the same, the existence is
presupposed of at least the following basic components,
constituting the basis of the invention:

1. A system of electrodes to pick up, at the cortical
level in a suitable region of the head, the signals outcoming from the player's brain, consisting of electrical
impulses of some prevailing frequencies comprised within
the range of from 5 to 35 cycles/second;

2. An amplifier system to the purpose of making electrically handleable the said picked-up signals;

3. An adequate system of filters, such as to sort the
useful band of variable electrical frequencies outcoming
from the brain and especially designed to eliminate those
undesirable frequencies such as e.g. the disturbing frequency of the feed network;

4. A system for the elimination of the amplitude disturbances due to more or less unconscious events from various, also motory muscular, origins;

5. A sensor system which originates encoded and compatible command impulses, according to what required by
the programes of the games, for the control of the competition, from the amplified cerebral electrical frequency
variations.

The components as indicated with the numbers 1, 2 and 3 are nowadays present in all encephalographs, or in all the equipment pieces designed for similar functions, with small technical variants, which however perfectly overcome the problems as briefly mentioned under the same numbers 2 and 3.

Adequate amplifiers, band filters, trapping filters for the ultra-low, lower than 5 cycles per second, frequencies, and for the frequencies higher than 30 cycles per second. Also active adjustable filters exist to eliminate the disturbing components deriving from the feeding voltage at network frequency, if the equipment is so fed. It must be outlined hence that even if to embody the present invention to a total extent, the use is absolutely necessary of these parts as hereinabove briefly described and already existing and well known, the two points 4 and 5 are instead introduced on purpose to accomplish our purpose, which is i.e. that of commanding the electronic games by means of voluntary expressions of the player's mind.

Rendering the encephalographic signals, of particular shape and value, suitable to be encoded and compatible with those mental systems for the commanding of the electronic games is the object and hence the only existence reason for the present invention.

In the enclosed schematic and exemplifying figure, the following component devices are shown in the order of the numbers: (1) Portion of helmet or of an equivalent item of light material, suitable to remain fixed on the player's head, without disturbing him; (2) electrodes of shapes adapted to render good and constant the picking up of the electrical systems outcoming from the brain;

their position on the head may be varied according to the material used and according to the point or the points selected for the picking-up. To this regard, the helmets used with the encephalographs already available on the markets are very important as examples to be considered. (3) High-gain amplifier, also of the differential type as already used, in the encephalographs, unless exemplified. (4) Low frequency eliminating filter. (5) High frequency eliminating filter. (6) A filter passing the band from 5 to 35 cycles per second, as already in use. (7) An amplitude eliminator for the electronic signals already amplified and deprived of any noises. The eliminator serves to eliminate amplitude increases which may sometimes be introduced thouth involuntary motory actions by the player's body, and finally (8) a transducer to prepare the signal which has to be encoded, as a function of the frequency variations present.

The block 9 serves to process the encoded signal, so as to make it compatible with the input 10 provided in the normal controls of the game, to interfere in the course of the same game, and as a replacement of the same.

Each function proposed in the various stages of the unit constituting the device substituent of the game manual controls, is a part of elementary and well-known electronic circuits, so that any engineer is capable to provide for the accomplishment or installation thereof. The whole unit, composed by the amplifier and by the various blocks, as hereinabove disclosed, may be constituted by a single piece presentable as an auxiliary element to be applied to the same electronic games by means of adapting means predisposed according to the various games types.

5.

The present invention can be rendered very versatile by means of the introduction of minor changes as desired so as to make the games more amusing and maybe more un-foreseeable.

0177075

C L A I M S

1. Electronic system prearranged to replace in the
electronic games the existing manual controls with
equivalent controls directly commanded by the brain,
characterized in that in it electrical signals outcoming
from the brain are used to command or to modify the
course of the electronic games, by one or more player(s).

2. Electronic system prearranged to replace in the electro
nic games the existing manual controls with equivalent
controls directly commanded by the brain, characterized
in that in it electrical signals are anyway used, which
can be obtained from other points of the human body to
constitute electrical actions compatible to be inserted into
the programmes of the electronic games, to the purpose of
replacing the manual controls already present, equally
altering, according to forecast schems, the outcomes of
the games.

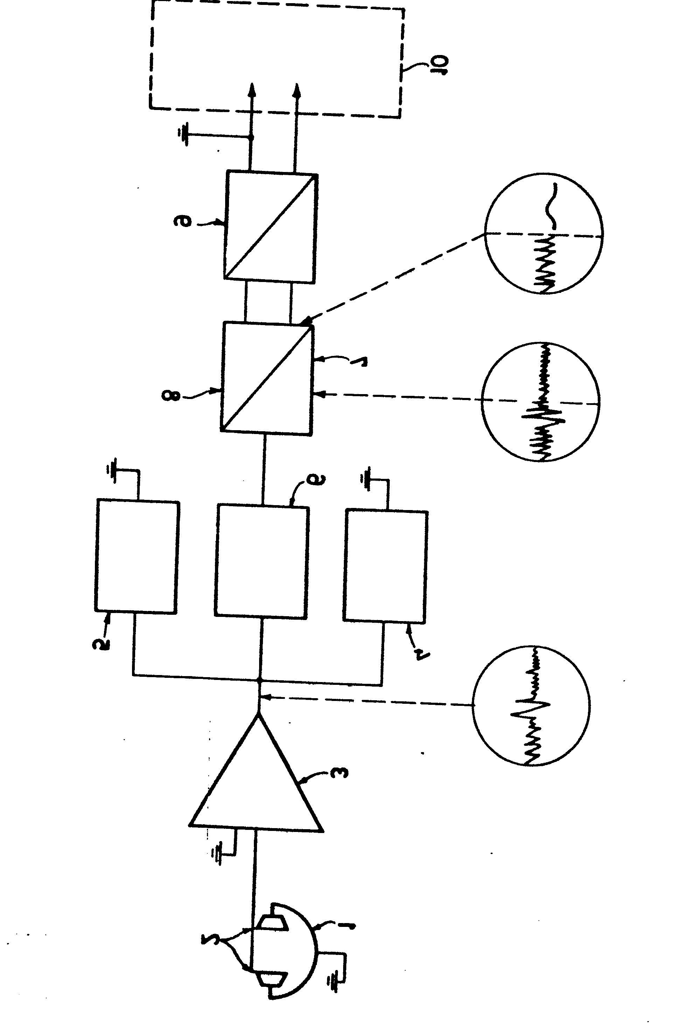

3. Electronic system prearranged to replace in the
electronic games the existing manual controls with equivalent controls directly commanded by the brain, characterized
in that in it the electrical signals picked-up at the
origin are then amplified, filtered and electronically
modified, until they become compatible, to be inserted
into the programme of the games and that to the purpose
as of claims 1 and 2.